(19) Europäisches Patentamt / European Patent Office / Office européen des brevets

(11) **EP 2 863 216 B1**

(12) **EUROPEAN PATENT SPECIFICATION**

(45) Date of publication and mention
of the grant of the patent:
**13.09.2017 Bulletin 2017/37**

(51) Int Cl.:
**G01N 30/86** *(2006.01)*     *G01N 30/88* *(2006.01)*
**G01N 33/28** *(2006.01)*

(21) Application number: **14189589.6**

(22) Date of filing: **20.10.2014**

(54) **Plus-fraction corrections for heavy hydrocarbon liquids**

Plus-Fraktionskorrekturen für schwere Kohlenwasserstoffflüssigkeiten

Corrections de fraction supplémentaire de liquides d'hydrocarbures lourds

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **18.10.2013 CA 2830384**

(43) Date of publication of application:
**22.04.2015 Bulletin 2015/17**

(73) Proprietor: **Weatherford Canada Partnership
Calgary, AB T2P 3B6 (CA)**

(72) Inventors:
• **Hu, YunFeng Y.**
 **Calgary, AB T3A 3W1 (CA)**
• **Thomas, F. Brent**
 **Crossfield, AB T0M 0S0 (CA)**

(74) Representative: **Suckling, Andrew Michael et al
Marks & Clerk LLP
Fletcher House
Heatley Road
The Oxford Science Park
Oxford OX4 4GE (GB)**

(56) References cited:
**WO-A1-2009/073269     WO-A2-2013/121204
US-A1- 2012 085 149**

• **JULIAN Y ZUO ET AL: "Plus Fraction
Characterization and PVT Data Regression for
Reservoir Fluids near Critical Conditions",
CONFERENCE INFO: SPE ASIA PACIFIC OIL AND
GAS CONFERENCE AND EXHIBITION, 16-18
OCTOBER, BRISBANE, AUSTRALIA, SOCIETY
OF PETROLEUM ENGINEERS, AU, 16 October
2000 (2000-10-16), pages 1-12, XP008167885,
DOI: 10.2118/64520-MS**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD

The present disclosure relates generally to analysis of heavy oil. More particularly, the present disclosure relates to chromatography of heavy oil.

### BACKGROUND

Chromatography includes the analysis of a compound to determine the identification and relative amount of the components. A sample of the compound is heated in a chromatographic column and the components detected at a detector.

Standards exist for the analysis of petroleum fractions including, for example, ASTM International standards relating to test methods for boiling range distribution of petroleum fractions by gas chromatography.

The quantification of heavy oil compositions is challenging for several reasons, which may include oxidation, emulsions, or impurities such as basic sediment and water (BS&W), or contamination.

However, one of the more difficult problems to resolve is the degree of non-elution, meaning the portion of the heavy oil that is not eluted, and thus does not arrive at the detector but instead remains behind in the chromatographic column.

It is, therefore, desirable to provide a method for chromatography of heavy oils.

WO 2009/073269 (Steinhecker) discloses methods and related apparatuses for gas chromatography using multiple detectors. A first detector is positioned to detect properties of a fluid at a location downstream of an injector and upstream of a first column. A second detector is positioned to detect properties of the fluid downstream from the first column. A processor receives and combines the measurement data from the first detector and the second detector to calculate a property associated with at least one of the components of the sample. In an embodiment disclosed, a quantity of fluid is injected as an injection plug, and the peak area of the injection plug is measured and used in the denominator of mass-fraction calculations (rather than the sum of eluted peak area) to yield a more accurate measure of the plus-fraction.

US 2012/085149 (Al-Eid et al.) discloses a method and apparatus for providing compositional analysis of hydrocarbon fluids from well samples up to a C30+ fraction. The sample is heated and thus separated into liquid and gaseous fractions. The respective liquid and gaseous fractions are analyzed by gas chromatography and the results combined to provide a total composition. In an embodiment disclosed, a plus-fraction defined as C30+, may be determined by iterative estimation and calculation.

WO 2013/121204 (Moorwood) discloses a method of characterizing a mixture comprising a plurality of hydrocarbons by gas chromatography. The method comprises obtaining a set of predetermined pseudo-components, each pseudo-component representing a respective group of hydrocarbons, obtaining physical data about the hydrocarbon mixture, and determining from the data the amount of each pseudo-component considered to be present in the mixture. Moorwood discloses estimating what is present in the plus fraction by regressing an empirical function to fit the measured data.

Zuo et al. "Plus Fraction Characterization and PVT Data Regression for Reservoir Fluids Near Critical Conditions", SPE Asia Pacific Oil and Gas Conference and Exhibition, Brisbane, Australia, October 16-18, 2000 (published SPE 64520) discusses plus fraction characterization involving distillation and modeling plus fractions, C7+, by fitting either an exponential or a three-parameter gamma distribution function.

### SUMMARY

The technique used for heavy oil characterization is based upon treatment of the heavy oil as two portions. A first portion includes components having a carbon number up to and including a full elution carbon number, and a second portion includes components having a carbon number greater than the full elution carbon number. In an embodiment disclosed the full elution carbon number is 60, and the first portion includes components having a carbon number up to and including $C_{60}$, and the second portion includes components having a carbon number $C_{60+}$. The first portion of the heavy oil may be characterized by typical means, for example gas chromatography. The second portion of the heavy oil may be characterized by the methods described herein. The characterization of the first portion and the second portion may be combined to provide a characterization for the total oil.

In an embodiment disclosed, a method for determining the area fraction of the heavy oil that is not eluted and, since area is proportional to mass in a flame ionization detector (FID), this technique provides the mass fraction non-eluted.

In a first aspect, the present disclosure provides a method of determining a plus-fraction correction for a heavy oil including obtaining an analysis of the heavy oil using gas chromatography to provide a highest detected carbon number ($C_{EMAX}$), a total eluted area ($A_{Etotal}$), and a $C_{60+}$ eluted area ($A_{EC60+}$), selecting an elution ratio (R) to compensate for the non-elution of a plus-fraction of the heavy oil, determining a corrected plus-fraction area ($A_{C60+}$) from the elution ratio (R) and the $C_{60+}$ eluted area ($A_{EC60+}$) according to the formula $A_{C60+} = A_{EC60+} \times R$, and determining a $C_{60}$ area ($A_{C60}$) and maximum carbon number ($C_{MAX}$) for the heavy oil according to the formulas $A_{EC60+} = 0.50 \times (C_{EMAX} - 60) \times A_{C60}$ and $A_{C60+} = 0.50 \times (C_{MAX} - 60) \times A_{C60}$. The analysis of the heavy oil using gas chromatography may, for example, include running a sample of the heavy oil thorough a gas chromatograph or using results from a previous gas chromatograph run for the heavy oil.

In an embodiment disclosed, the elution ratio (R), is determined according to the formula $R = 0.0493 \ln (A_{C60+} A_{Etotal} \times 100) + 1.1746$.

In an embodiment disclosed, the method further includes determining a slope of a non-elution line according to the formula slope $= (0 - A_{C60}) / (C_{MAX} - 60)$, and determining at least one area fraction for a carbon number ($C_{Ni}$) according to the formula $A_{CNi} = A_{C60} + slope \times (C_{Ni} - 60)$.

In an embodiment disclosed, the method further includes determining an area fraction $A_{CNi}$, for each carbon number ($C_{Ni}$) between $C_{60}$ and $C_{MAX}$.

In an embodiment disclosed, the method further includes determining a mole fraction for each carbon number ($C_{Ni}$) between $C_{60}$ and $C_{MAX}$.

In an embodiment disclosed, the method further includes summing the molecular weight fraction for each carbon number ($C_{Ni}$) between $C_{60}$ and $C_{MAX}$ to determine a $C_{60+}$ molecular weight ($MW_{C60+}$) of the heavy oil.

In an embodiment disclosed, the method further includes summing the molecular weight fraction for each carbon number ($C_{Ni}$) between $C_{90}$ and $C_{MAX}$ to determine a $C_{90+}$ molecular weight ($MW_{C90+}$) for the heavy oil.

In an embodiment disclosed, the method further includes summing the molecular weight fraction for each carbon number ($C_{Ni}$) between $C_{MIN}$ and $C_{MAx}$ to determine a molecular weight (MW) of the heavy oil.

In an embodiment disclosed, $C_{MIN}$ equals $C_3$.

In an embodiment disclosed, the method further includes determining a plus-fraction area, between $C_{60}$ and $C_{MAX}$, according to the formula $A_{C60+} = \int A_{CNi} * dC_N$.

In an embodiment disclosed, the method further includes determining a plus-fraction area, between $C_{60}$ and $C_{MAX}$, according to the formula $A_{C60+} = A_{C60} \times C_{Ni} + slope (C_{Ni}^2/2 - 60 \times C_{Ni})$.

In an embodiment disclosed, $A_{C60+} / A_{Etotal} \times 100$ is between 1 percent and 100 percent. In an embodiment disclosed, $A_{C60+} / A_{Etotal} \times 100$ is between 2 percent and 40 percent. In an embodiment disclosed, $A_{C60+} / A_{Etotal} \times 100$ is between 2 percent and 30 percent.

A computer may be utilized to make the calculations of the present disclosure. In an embodiment, a computer-readable medium has computer-readable code embodied therein. The computer-readable code is executable by a processor to use an analysis of the heavy oil using gas chromatography to provide a highest detected carbon number ($C_{EMAX}$), a total eluted area ($A_{Etotal}$), and a $C_{60+}$ eluted area ($A_{EC60+}$), and selecting an elution ratio (R) to compensate for the non-elution of a plus-fraction of the heavy oil, automatically determining a corrected plus-fraction area ($A_{C60+}$) from the elution ratio (R) and the $C_{60+}$ eluted area ($A_{EC60+}$) according to the formula $A_{C60+} = A_{EC60+} \times R$, and determining a $C_{60}$ area ($A_{C60}$) and maximum carbon number ($C_{MAX}$) for the heavy oil according to the formulas $A_{EC60+} = 0.50 \times (C_{EMAX} - 60) \times A_{C60}$ and $A_{C60+} = 0.50 \times (C_{MAX} - 60) \times A_{C60}$.

Other aspects and features of the present disclosure will become apparent to those ordinarily skilled in the art upon review of the following description of specific embodiments in conjunction with the accompanying figures.

## BRIEF DESCRIPTION OF THE DRAWINGS

Embodiments of the present disclosure will now be described, by way of example only, with reference to the attached Figures.

Fig. 1 is a sample chromatogram for a heavy oil sample;
Fig. 2 is a correlation of an elution ratio R versus percent $C_{60+}$ eluted;
Fig. 3 is a detail of the $C_{60+}$ portion of Fig. 1, illustrating the relationship between an area eluted for $C_{60+}$ ($A_{EC60+}$) and an area non-eluted for $C_{60+}$ ($A_{NEC60+}$); and
Fig. 4 is a table for an example of the present disclosure.

## DETAILED DESCRIPTION

Generally, the present disclosure provides a method and apparatus for quantification of heavy oil which compensates for the non-elution of a portion of the heavy oil being tested.

**Chromatography**

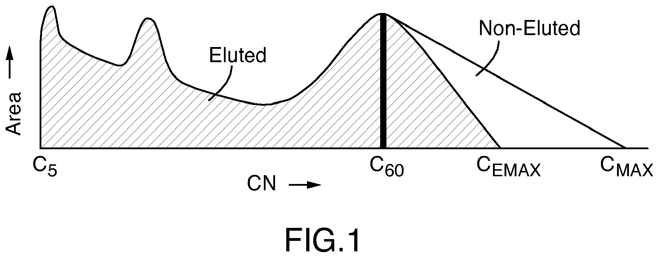

Referring to Fig. 1, chromatography provides identification and quantification of the components of a compound, for example carbon atoms in heavy oil. The relative amount of each component, for example $C_{MIN}$ through $C_{EMAX}$ is indicated by the area of each component. The $C_{MIN}$ compound being the compound with the lowest carbon number eluted that registers a non-zero amount on the chromatograph. $C_{MIN}$ may be typically about $C_5$, as shown in Fig. 1 for a heavy oil at atmospheric conditions. The compound $C_{EMAX}$ being the compound with the highest carbon number eluted that registers a non-zero amount on the chromatograph. Components undoubtedly exist beyond $C_{EMAX}$, for example up to and including $C_{MAX}$, but are not eluted and therefore are not revealed by the chromatograph.

On the basis of poly-wax quantitative sample containing 16 components up to $C_{60}$, it is understood that using a high temperature gas chromatograph which operates up to 400°C and uses helium as a carrier gas, that 100 percent elution is achieved at least up to and including $C_{60}$.

However, non-elution may start at $C_{61}$ or beyond but no quantitative sample exists where the degree of elution can be quantified. Therefore, in an embodiment disclosed, the full elution carbon number, being the highest carbon number where substantially one-hundred (100) percent elution is known to be achieved, is selected as $C_{60}$.

**Elution Ratio R**

In an embodiment disclosed, a method for determining the area fraction of the heavy oil non-eluted is provided, and since area is proportional to mass in a flame ionization detector (FID), this technique provides the mass fraction non-eluted.

In an embodiment disclosed, a non-eluted correction may be expressed as:

$$A_{NEC60+} = A_{EC60+} \, (R - 1) \qquad\qquad \text{Eq. (1)}$$

In Eq. (1), $A_{NEC60+}$ is the area of the $C_{60+}$ not eluted and $A_{EC60+}$ is the area of the eluted $C_{60+}$. The elution ratio, R is equal to the total amount of hydrocarbons ($A_{EC60+}$ plus $A_{NEC60+}$) over the amount of hydrocarbons eluted ($A_{EC60+}$):

$$R = (A_{EC60+} + A_{NEC60+}) \, / \, A_{EC60+} \qquad\qquad \text{Eq. (2)}$$

Eq. (2) may be rewritten by substituting the total area $A_{C60+}$ for the area eluted $A_{EC60+}$ plus the area non-eluted $A_{NEC60+}$:

$$R = A_{C60+} \, / \, A_{EC60+} \qquad\qquad \text{Eq. (3)}$$

The elution ratio, R, may be represented as:

$$R = C_{60+} \text{ Total} \, / \, C_{60+} \text{ Eluted} \qquad\qquad \text{Eq. (4)}$$

**Determination of Elution Ratio, R**

The $C_{60+}$ total area was derived from multiple blank runs performed after a sample of heavy oil was provided into the gas chromatograph column. The total area of the blanks cumulated and added to the eluted $C_{60+}$ area divided by the eluted $C_{60+}$ provides an elution ratio, R.

As an example, a heavy oil of known composition, labeled C-E006 was sampled in the gas chromatograph. This sample indicated an eluted $C_{60+}$ of 3.69569, but was known to have a total $C_{60+}$ of 5.3272, so a non-eluted $C_{60+}$ of 1.6315 may be determined. Applying Eq. (4) provides R = 1.441.

In an embodiment disclosed, a correlation is provided to determine and use the elution ratio, R for a sample heavy oil of unknown composition.

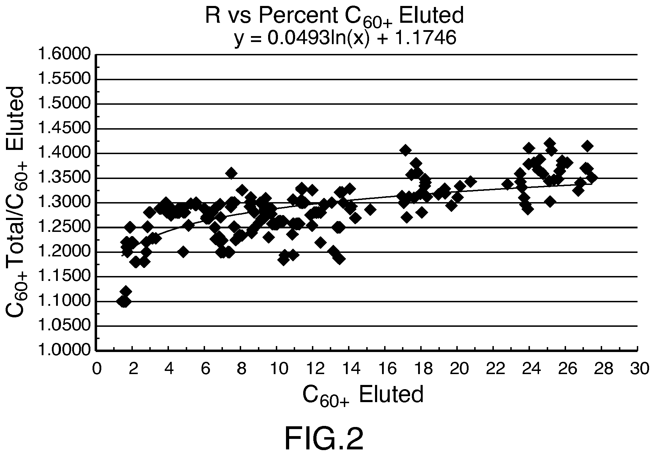

Referring to Fig. 2, the elution ratio, R is shown in correlation with percent $C_{60+}$ eluted for wide variety of heavy oils, that is percent $C_{60+}$ eluted = $A_{EC60+}$ / $A_{Etotal}$ x 100. In an embodiment disclosed, the logarithmic curve fit correlation is represented as:

$$R = 0.0493 \ln (\text{percent } C_{60+} \text{ Eluted}) + 1.1746 \qquad \text{Eq. (5)}$$

A lower limit of 0.03 percent $C_{60+}$ may be used, in order to limit the R to 1.00, i.e., using Eq. (5) 0.0493 ln (0.03) + 1.1746 equals substantially 1.00.

With a sample of heavy oil of unknown composition, one can conduct a gas chromatograph run, and using the percent $C_{60+}$ eluted ($A_{EC60+}$ / $A_{Etotal}$ *100), determine the elution ratio, R, for the sample. The elution ratio, R determined in this manner may be used to compensate for the non-elution.

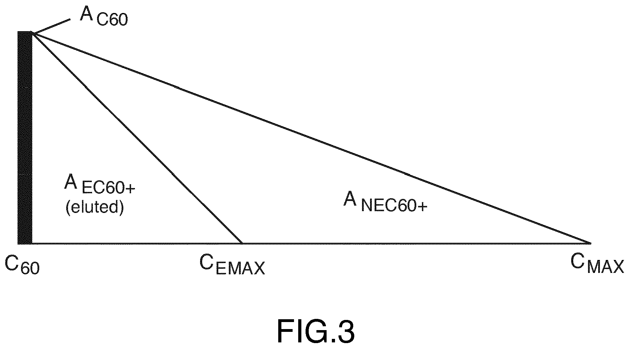

Referring to Fig. 3, a detail of the chromatograph of Fig. 1, from $C_{60}$ and higher is shown.

With the elution ratio, R and utilizing a linear elution phenomenon in the range of the components in question, $C_{60}$ through $C_{EMAX}$, and $C_{60}$ through and $C_{MAX}$, two linear equations result, where the area of the $C_{60}$ component is determined and subsequently the maximum carbon number (CN) of the non-eluted fraction is determined.

**$A_{C60}$**

Using the $A_{EC60+}$ value and $C_{EMAX}$ value measured from the heavy oil sample using the gas chromatograph, the area for the $C_{60}$ component, being $A_{C60}$, may be determined from:

$$A_{EC60+} = 0.50 \times (C_{EMAX} - 60) \times A_{C60} \qquad \text{Eq. (6)}$$

**$C_{MAX}$**

Then $A_{C60+}$ may be determined using R and $A_{EC60+}$ using Eq. 3, from above. Finally, $C_{MAX}$ may be calculated using the $A_{C60+}$ and $A_{C60}$ values from above, and equation (7) the maximum carbon number (CN) $C_{MAX}$ of the non-eluted fraction:

$$A_{C60+} = 0.50 \times (C_{MAX} - 60) \times A_{C60} \qquad \text{Eq. (7)}$$

**Components**

The equation of the gas chromatograph area for each component, including eluted and non-eluted contributions, may be derived, and from that the total area of the $C_{60+}$ may be determined.

Knowing the mass fraction for every component in the $C_{60+}$ range, and assuming a molecular weight (MW) increment of 14.0267 per carbon number (C-$H_2$), as in an alkane $C_nH_{2n+2}$, the renormalized $C_{60+}$ can be used to compute the $C_{60+}$ molecular weight.

The total $A_{C60+}$ is determined by integrating the area over the range of carbon numbers in the range.

**Example 1: Heavy Oil Sample WL1**

Referring to Figs. 1, and 3, in an example, a heavy oil sample WL1 is analyzed using a high temperature gas chromatograph column, which operates at up to 400 °C and uses helium as a carrier gas. The full elution carbon number is selected as 60, that is, $C_{60}$.

The gas chromatograph indicates the presence of hydrocarbons up to $C_{108}$. That is, the highest degree carbon component showing any significant degree of detected elution is $C_{108}$. Therefore $C_{EMAX}$=108. The chromatograph further indicates that the $A_{EC60+}$ = 1,908,000 (area for eluted $C_{60+}$) and that $A_{Etotal}$ = 14,290,000 (total area).

**Elution Ratio, R**

In an embodiment, disclosed is a correlation between the elution ratio R, and the eluted $C_{60+}$ area $A_{EC60+}$ relative to the eluted total area $A_{Etotal}$. In this example, $A_{EC60+}$ / $A_{Etotal}$ = 1,908,000 / 14,290,000 = 0.1335 = 13.35 percent.

Referring to Fig. 2, or Eq. (5), 13.35 percent provides an elution ratio, R of = 0.0493 ln (13.35) + 1.1746 = 1.3024.

Using the $A_{EC60+}$ of 1,908,000 measured from the gas chromatograph and the elution ratio R = 1.3024, $A_{C60+}$ is determined according to Eq. (3), which may be rearranged:

$$A_{C60+} = A_{EC60+} \times R \qquad\qquad \text{Eq. (8)}$$

Solving this equation provides $A_{C60+}$ = 1,908,000 x 1.3024 = 2,484,979, which as above, is the total area of eluted and non-eluted for $C_{60+}$.

**Quantification**

Using Eq. (6) above with the $A_{EC60+}$ and $C_{EMAX}$ data from the chromatograph provides the area of the $C_{60}$ component:

$$1{,}908{,}000 = 0.50 \times (108 - 60) \times A_{C60} \qquad\qquad \text{from Eq. (6)}$$

In this example, with $A_{EC60+}$ = 1,908,000 and $C_{EMAX}$ = 108 from the chromatograph, Eq. (6) provides $A_{C60}$ = 79,500.0.
$C_{MAX}$ is determined according to Eq. (7) above using $A_{C60+}$ from Eq. (8):

$$2{,}484{,}979 = 0.50 \times (C_{MAX} - 60) \times 79{,}500 \qquad\qquad \text{from Eq. (7)}$$

Solving Eq. (7) with $A_{C60+}$ = 2,484,979 (this is the $A_{C60+}$ based on the elution ratio, R) and $A_{C60}$ = 79,500.0, provides $C_{MAX}$ = 122.52. As the carbon number (CN) must be an integer, $C_{MAX}$ is rounded up to the next higher integer. The calculated $C_{MAX}$ = 122.52 is thus rounded up to $C_{MAX}$ = 123.

**Corrected Profile**

Referring to Fig. 3, the slope of the non-eluted line is:

$$\text{Slope} = (0 - A_{C60}) / (C_{MAX} - 60) \qquad\qquad \text{Eq. (9)}$$

As the values for $A_{C60}$, $C_{MAX}$, and $C_{EMAX}$ are known, Eq. (9) can be solved:

$$\text{Slope} = (0 - 79500.0) / (123 - 60) = -1261.9048 \qquad\qquad \text{from Eq. (9)}$$

The slope may then be used to determine the actual area for any component from $C_{60}$ through $C_{MAX}$, which takes into account the elution ratio, R, and thus compensates for the non-elution of the heavy oil:

$$A_{CNi} = A_{C60} + \text{Slope} \times (C_{Ni} - 60) \quad \text{Eq. (10)}$$

$$A_{CNi} = 79{,}500 - 1{,}261.9048 \times (C_{Ni} - 60) \qquad\qquad \text{from Eq. (10)}$$

For example, the area $A_{C100}$ for $C_{100}$ equals, according to Eq. (7):

$$79{,}500 - 1{,}261.9048 \times (100 - 60) = 29{,}023.8 \qquad\qquad \text{from Eq. (10)}$$

This may be calculated between 0 to $C_{MAX}$). The equation of the gas chromatograph area for each component, including eluted and non-eluted contributions, is derived from which the total area of the $C_{60+}$ is readily available. This calculation can be performed for any of CNi, where in this case i is between 60 and $C_{MAX}$ (in this example $C_{MAX}$ = 123) to provide the area, and thus mass fraction, or mole fraction, or both for any component.

**Mass Fraction (MF)**

One can also sum, totalize, or integrate the area $A_{CNi}$ from $C_{60}$ to $C_{MAX}$, to determine the total area:

$$A_{C60+} = \int A_{CNi} * dC_N, \text{ between } C_{60} \text{ and } C_{MAX}. \qquad \text{Eq. (11)}$$

Using Eq. (10) above and performing the integration, Eq. (11), provides the formula:

$$A_{C60+} = A_{C60} \times C_{Ni} - 1261.9048 \, (C_{Ni}^2/2 - 60 \times C_{Ni}) \qquad \text{Eq. (12)}$$

Calculating $A_{C60+}$ with $C_{Ni}$ between 60 and $C_{MAX}$, provides $A_{C60+}$ = 2,504,249.92.

There is a small difference between the $A_{C60+}$ of 2,504,249.92 calculated here and the $A_{C60+}$ of 2,484,979 calculated at Eq. (8) due to the rounding up of the $C_{MAX}$ to an integer, in this example from 122.52 to 123.

Using the $A_{Etotal}$ above from the gas chromatograph, the mass fraction of $C_{60+}$ ($MF_{C60+}$) may be determined:

$$MF_{C60+} = A_{C60+} / (A_{Etotal} + A_{NEC60+}) \qquad \text{Eq. (13)}$$

Using the $A_{C60+}$ calculated above, the $A_{Etotal}$ from the gas chromatograph, and using the $A_{NEC60+} = A_{EC60+} (R - 1)$ from Eq. (1), the mass fraction of $C_{60+}$ is:

$$MF_{C60+} = A_{C60+} / (A_{Etotal} + A_{EC60+} \times (R-1)) \qquad \text{from Eq. (13)}$$

Using $A_{NEC60+} = A_{EC60+} (R - 1)$ from equation (1), and the previously determined $A_{EC60+}$ and R, solving Eq. (13) provides:

$$MF_{C60+} = 2,504,249.92 / (14,290,000 + 1,908,000 \times (1.3024 - 1))$$

$$MF_{C60+} = 0.1684 \qquad \text{from Eq. (13)}$$

This indicates that, in this example, the mass fraction for $C_{60+}$ is 0.168.

**Molecular Weight**

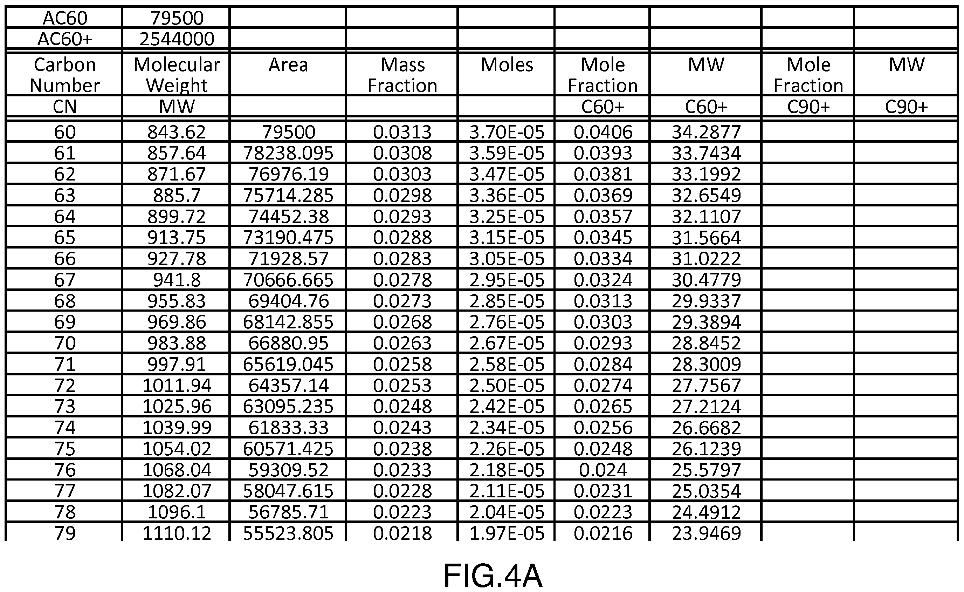

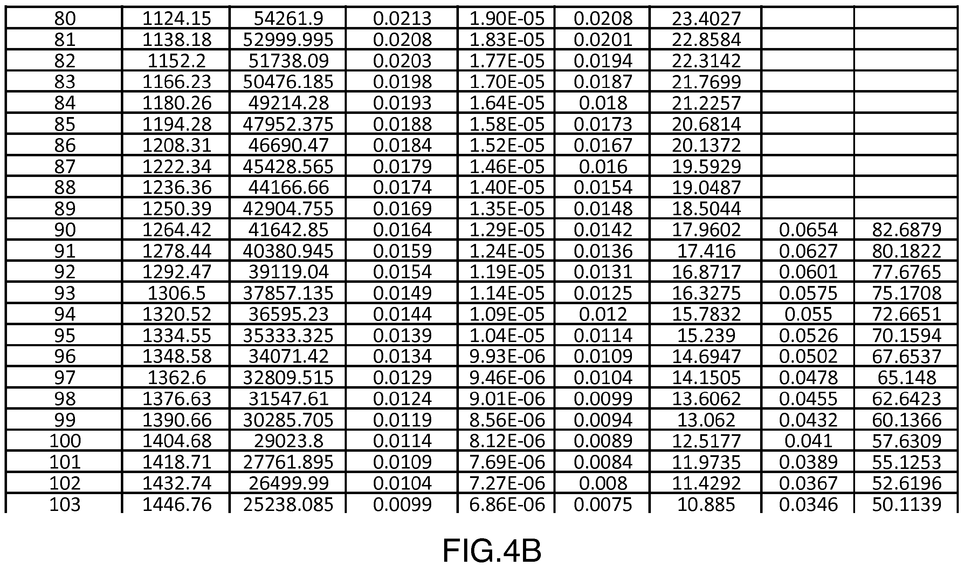

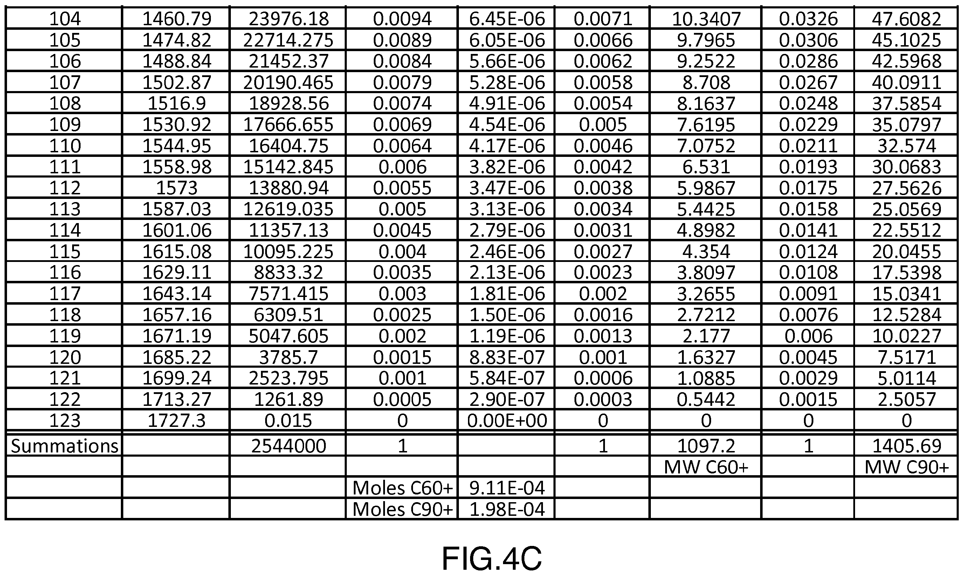

Referring to Fig. 4, a table provides an example determination of the molecular weight $C_{60+}$ ($MW_{C60+}$) and molecular weight $C_{90+}$ ($MW_{C90+}$) using the methods described herein. Such quantifications are commonly used for calculations or analysis, for example computer simulations of heavy oil reservoirs.

For each carbon number, $C_{Ni}$, the molecular weight, area, mass fraction, moles, $C_{60+}$ mole fraction, and $C_{90+}$ mole fraction may be determined.

It is assumed that the hydrocarbon is an alkane, i.e. $C_nH_{2n+2}$. Using a molecular weight of C of 12.01078 g/mol and a molecular weight of H of 1.007947 g/mol, for example, $C_{100}$, as $C_{100}H_{202}$, would have a molecular weight of 100 x 12.01078 + 202 * 1.007944 = 1,404.68 g/mol.

The area for $C_{100}$, as $A_{C100}$ may also be calculated, as above, and as shown in Fig. 4, $A_{C100}$ is 29,023.8.

The areas for each of $C_{Ni}$ may be totaled. In Fig. 4, the total area for $C_{60}$ though $C_{123}$ is 2,504,250. Again, there is a small difference between the $A_{C60+}$ of 2,504,250 calculated here and the $A_{C60+}$ of 2,504,250 and the $A_{C60+}$ of 2,484,979 calculated earlier.

For each $C_{Ni}$, the mass fraction may be determined from the area divided by the total area. In the example, $C_{100}$, having an area $A_{C100}$ of 29,023.8 divided by the total area of 2,544,000 provides the mass fraction of $C_{100}$. As shown the mass fraction of $C_{100}$ is 29,023.8 / 2,544,000 = 0.0114. The number of moles of $C_{100}$ is the mass fraction divided by the molecular weight, that is 0.0114 / 1,404.68.45 = 8.1219 x $10^{-6}$. This is repeated for each $C_{Ni}$ between $C_{60}$ through $C_{MAX}$.

The number of moles for $C_{60+}$ and $C_{90+}$ may be summed. As shown in Fig. 4, the total $C_{60+}$ is 9.114 x $10^{-4}$ and the total for $C_{90+}$ is 1.9796 x $10^{-4}$.

The mole fraction may be calculated for each $C_{Ni}$, for each of $C_{60+}$ and $C_{90+}$.

As shown in the example in Fig. 4, the mole fraction for $C_{100}$ is 0.0089 for $C_{60+}$ and 0.0410 for $C_{90+}$.

The mole fraction for each $C_{Ni}$ may be multiplied by the molecular weight, and the product sum provides the molecular weight. In the example shown in Fig. 4, the molecular weight of the $C_{60+}$ components is 1,097.20 g/mol, and the molecular weight of the $C_{90+}$ components is 1,405.69 g/mol.

**Total Oil**

The composition of the whole oil may be provided using the complete elution of the $C_5$ to $C_{59}$ components from the gas chromatograph combined with the $C_{60+}$ as determined in accordance with the present disclosure.

**Subsequent applications of determined material properties**

The corrected plus fraction and/or molecular weight and/or mole fraction data may be used by an entity such as an oil company to determine material properties of the heavy oil with improved accuracy. The corrected plus fraction and/or molecular weight and/or mole fraction may also be used in a computer simulation or in modeling software to better predict heavy oil production or heavy oil reserves. An entity may then use such a simulation or model, benefitting from the more accurately determined material properties, to design equipment for conveying and processing the heavy oil, such equipment including but not limited to: pumps, piping, pipelines, separators, fractionators, catalytic crackers, and other equipment to produce, convey, treat or refine the heavy oil.

Accurate knowledge of the composition may also be important for refining and marketing the oil. For example, an entity may represent to a pipeline operator or refinery what the composition of the heavy oil is for pricing or refining. Because of non-elution of the heavy oil other methods may not provide sufficient accuracy, which can be important for such uses of the oil.

The corrected molecular weight and/or mole fraction and/or other properties of the heavy oil may be used in the ways that a person skilled in the art would use such material properties. In the case of heavy oil, the non-elution can be significant. In some heavy oils, up to 50% of the heavy oil can be lost to non-elution, meaning that conventional techniques provide less accurate determination of the material properties. The correction techniques disclosed may be very significant to obtaining accurate data.

**General**

The technique can easily be generalized to provide the molecular weight and mass fraction for a selected plus fraction.

As used herein, heavy oil is defined as petroleum liquids having an API gravity of less than about 12 °API (i.e. a density of greater than 986 kg/m$^3$).

According to another aspect of the present disclosure, the quantification may be made using another selected plus-fraction, for example $C_{90+}$ or another selected plus-fraction. As an example, another selected plus-fraction may be $C_{50+}$, $C_{120+}$ or $C_{100+}$ or some other selected plus-fraction, in which case the selected plus-fraction would be substituted in the above description for $C_{60+}$. That is, for example, the selected cutoff could be at other than $C_{60}$, with corresponding adjustments in the above method.

In the preceding description, for purposes of explanation, numerous details are set forth in order to provide a thorough understanding of the embodiments. However, it will be apparent to one skilled in the art that these specific details are not required.

The above-described embodiments are intended to be examples only. Alterations, modifications and variations can be effected to the particular embodiments by those of skill in the art without departing from the scope, which is defined solely by the claims appended hereto. Each feature disclosed or illustrated in the present specification may be incorporated in the invention, whether alone or in any appropriate combination with any other feature disclosed or illustrated herein.

**Claims**

1. A method of determining a plus-fraction correction for a heavy oil comprising:

   obtaining an analysis of the heavy oil using gas chromatography to provide a highest detected carbon number ($C_{EMAX}$), a total eluted area ($A_{Etotal}$), and an eluted area ($A_{EC60+}$) for carbon numbers greater than and equal to 60 ($C_{60+}$);
   selecting an elution ratio (R) to compensate for the non-elution of a plus-fraction of the heavy oil;

determining a corrected plus-fraction area ($A_{C60+}$) from the elution ratio (R) and the $C_{60+}$ eluted area ($A_{EC60+}$) according to the formula $A_{C60+} = A_{EC60+} \times R$; and

determining a $C_{60}$ area ($A_{C60}$) and maximum plus-fraction carbon number ($C_{MAX}$) for the heavy oil according to the formulas $A_{EC60} + = 0.50 \times (C_{EMAX} - 60) \times A_{C60}$ and $A_{C60+} = 0.50 \times (C_{MAX} - 60) \times A_{C60}$,

wherein said obtaining an analysis of the heavy oil using gas chromatography comprises running a sample of the heavy oil thorough a gas chromatograph or using results from a previous gas chromatograph run for the heavy oil.

2. The method of claim 1, wherein the elution ratio (R) is determined according to the formula $R = 0.0493 \ln (A_{C60+} / A_{Etotal} \times 100) + 1.1746$.

3. The method of claim 2, further comprising:

determining a slope of a non-elution line according to the formula slope = $(0 - A_{C60}) / (C_{MAX} - 60)$; and

determining at least one area fraction for a carbon number ($C_{Ni}$) according to the formula $A_{CNi} = A_{C60} + $ slope $\times (C_{Ni} - 60)$.

4. The method of claim 3, further comprising determining an area fraction $A_{CNi}$, for each carbon number ($C_{Ni}$) between $C_{60}$ and $C_{MAX}$.

5. The method of claim 4, further comprising determining a mole fraction for each carbon number ($C_{Ni}$) between $C_{60}$ and $C_{MAX}$.

6. The method of claim 5, further comprising summing the molecular weight fraction for each carbon number ($C_{Ni}$) between $C_{60}$ and $C_{MAX}$ to determine a $C_{60+}$ molecular weight (MWC60+) of the heavy oil.

7. The method of claim 6, further comprising summing the molecular weight fraction for each carbon number ($C_{Ni}$) between $C_{90}$ and $C_{MAX}$ to determine a molecular weight ($MW_{C90+}$) for carbon numbers greater than and equal to 90 ($C_{90+}$) for the heavy oil.

8. The method of claim 5, further comprising summing the molecular weight fraction for each carbon number ($C_{Ni}$) between $C_{MIN}$ and $C_{MAX}$ to determine a molecular weight (MW) of the heavy oil.

9. The method of claim 8, where $C_{MIN}$ equals $C_3$.

10. The method of claim 2, wherein $A_{C60+} / A_{Etotal} \times 100$ is between 1 percent and 100 percent, preferably between 2 percent and 40 percent or more preferably between 2 percent and 30 percent.

11. The method of claim 5, wherein the mole fractions are used to provide material properties for the heavy oil for design of equipment for conveying or processing the heavy oil.

12. The method of claim 7, wherein the $MW_{C60+}$ and the $MW_{C90+}$ are provided as inputs to a computer model to provide heavy oil material properties for simulating heavy oil recovery from a heavy oil reservoir containing the heavy oil.

13. The method of claim 8, wherein the molecular weight (MW) is used to provide material properties for the heavy oil for design of equipment for conveying or processing the heavy oil.

14. A computer-readable medium having computer-readable code embodied therein, wherein the computer-readable code when executed by a processor of a computing device implements the method according to any one of claims 1 to 13.

15. An apparatus arranged to determine a plus-fraction correction for a heavy oil, the apparatus being configured to perform the method of any of claims 1 to 13.

**Patentansprüche**

1. Verfahren zur Bestimmung einer Plus-Fraktionskorrektur für ein Schweröl, folgende Schritte beinhaltend:

Erzielen einer Analyse des Schweröls unter Verwendung von Gaschromatographie zur Bereitstellung einer höchsten nachgewiesenen Kohlenstoffzahl ($C_{EMAX}$), eines eluierten Gesamtbereichs ($A_{Etotal}$), und eines eluierten Bereichs ($A_{EC60+}$) für Kohlenstoffzahlen größer als und gleich 60 ($C_{60+}$);

Auswählen eines Elutionsverhältnisses (R) zur Kompensierung der Nichtelution einer Plus-Fraktion des Schweröls;

Bestimmen eines korrigierten Plus-Fraktionsbereichs ($A_{C60+}$) anhand des Elutionsverhältnisses (R) und des eluierten $C_{60+}$-Bereichs ($A_{EC60+}$) nach der Formel $A_{C60+} = A_{EC60+} \times R$; und

Bestimmen eines $C_{60}$-Bereichs ($A_{C60}$) und einer maximalen Plus-Fraktions-Kohlenstoffzahl ($C_{MAX}$) für das Schweröl nach den Formeln $A_{EC60+} = 0{,}50 \times (C_{EMAX}-60) \times A_{C60}$ und $A_{C60+} = 0{,}50 \times (C_{MAX} - 60) \times A_{C60}$,

wobei das Erzielen einer Analyse des Schweröls unter Verwendung von Gaschromatographie das Testen einer Probe des Schweröls in einem Gaschromatografen beinhaltet, oder die Verwendung von Ergebnissen einer zuvor für Schweröl bewerkstelligten Gaschromatografie.

2. Verfahren nach Anspruch 1, bei welchem das Elutionsverhältnis (R) nach der Formel $R = 0.0493 \ln (A_{C60+} / A_{Etotal} \times 100) + 1.1746$ bestimmt wird.

3. Verfahren nach Anspruch 2, weiterhin beinhaltend:

Bestimmen einer Steilheit einer Nichtelutionskurve nach der Formel Steilheit = $(0 - A_{C60}) (C_{MAX} - 60)$; und

Bestimmen von mindestens einer Bereichsfraktion für eine Kohlenstoffzahl ($C_{Ni}$) nach der Formel $A_{CNi} = A_{C60}$ + Steilheit $\times (C_{Ni} - 60)$.

4. Verfahren nach Anspruch 3, weiterhin beinhaltend das Bestimmen einer Bereichsfraktion $A_{CNi}$ für jede Kohlenstoffzahl ($C_{Ni}$) zwischen $C_{60}$ und $C_{MAX}$.

5. Verfahren nach Anspruch 4, weiterhin beinhaltend das Bestimmen einer Molfraktion für jede Kohlenstoffzahl ($C_{Ni}$) zwischen $C_{60}$ und $C_{MAX}$.

6. Verfahren nach Anspruch 5, weiterhin beinhaltend das Addieren der Molekulargewichtsfraktion für jede Kohlenstoffzahl ($C_{Ni}$) zwischen $C_{60}$ und $C_{MAX}$ zur Bestimmung eines $C_{60+}$-Molekulargewichts (MWC60+) des Schweröls.

7. Verfahren nach Anspruch 6, weiterhin beinhaltend das Addieren der Molekulargewichtsfraktion für jede Kohlenstoffzahl ($C_{Ni}$) zwischen $C_{90}$ und $C_{MAX}$ zur Bestimmung eines Molekulargewichts ($MW_{C90+}$) für Kohlenstoffzahlen größer als 90 ($C_{90+}$) des Schweröls.

8. Verfahren nach Anspruch 5, weiterhin beinhaltend das Addieren der Molekulargewichtsfraktion für jede Kohlenstoffzahl ($C_{Ni}$) zwischen $C_{MIN}$ und $C_{MAX}$ zur Bestimmung eines Molekulargewichts (MW) des Schweröls.

9. Verfahren nach Anspruch 8, bei welchem $C_{MIN}$ gleich $C_3$ ist.

10. Verfahren nach Anspruch 2, bei welchem $A_{C60}$, / $A_{Etotal}$ $\times$ 100 zwischen 1 Prozent und 100 Prozent beträgt, vorzugsweise zwischen 2 Prozent und 40 Prozent oder noch bevorzugter Weise zwischen 2 Prozent und 30 Prozent.

11. Verfahren nach Anspruch 5, bei welchem die Molfraktionen verwendet werden, um Materialeigenschaften des Schweröls zur Konstruktion von Ausrüstungen zur Beförderung oder Verarbeitung des Schweröls bereitzustellen.

12. Verfahren nach Anspruch 7, bei welchem das $MW_{C60+}$ und das $MW_{C90+}$ als Eingabedaten in ein Computermodell bereitgestellt werden, um Schweröl-Materialeigenschaften zur Simulation von Schwerölrückgewinnung aus einem das Schweröl enthaltenden Schweröltank bereitzustellen.

13. Verfahren nach Anspruch 8, bei welchem das Molekulargewicht (MW) verwendet wird, um Materialeigenschaften des Schweröls zur Konstruktion von Ausrüstungen zur Beförderung oder Verarbeitung des Schweröls bereitzustellen.

14. Computerlesbares Medium, besitzend einen hierin eingebetteten computerlesbaren Code, wobei der computerlesbare Code bei der Ausführung durch einen Prozessor eines Rechengerätes das Verfahren nach einem der Ansprüche 1 bis 13 implementiert.

**15.** Gerät, geeignet zur Bestimmung einer Plus-Fraktionskorrektur für ein Schweröl, wobei das Gerät konfiguriert ist, das Verfahren nach einem der Ansprüche 1 bis 13 zu bewerkstelligen.

**Revendications**

**1.** Procédé de détermination d'une correction de fraction supplémentaire pour du pétrole lourd, comprenant :

l'obtention d'une analyse du pétrole lourd en utilisant une chromatographie en phase gazeuse de manière à obtenir un nombre de carbones détectés le plus élevé ($C_{EMAX}$), une aire éluée totale ($A_{Etotal}$) et une aire éluée ($A_{EC60+}$) pour des nombres de carbone supérieurs et égaux à 60 ($C_{60+}$) ;
la sélection d'un taux d'élution (R) de manière à compenser la non élution d'une fraction supplémentaire du pétrole lourd ;
la détermination d'une aire de fraction supplémentaire corrigée ($A_{C60+}$) à partir du taux d'élution (R) et de l'aire éluée de $C_{60+}$ ($A_{EC60+}$) conformément à la formule $A_{C60+} = A_{EC60+} \times R$ ; et
la détermination d'une aire de $C_{60}$ ($A_{C60}$) et d'un nombre de carbones de fraction supplémentaire maximum ($C_{MAX}$) pour le pétrole lourd conformément aux formules $A_{EC60+} = 0{,}50 \times (C_{EMAX} - 60) \times A_{C60}$ et $A_{C60+} = 0{,}50 \times (C_{MAX} - 60) \times A_{C60}$,
dans lequel ladite obtention d'une analyse du pétrole lourd en utilisant une chromatographie en phase gazeuse comprend le fait de faire passer un échantillon du pétrole lourd au travers d'un chromatographe en phase gazeuse ou le fait d'utiliser des résultats qui proviennent d'une passe précédente en chromatographe en phase gazeuse pour le pétrole lourd.

**2.** Procédé selon la revendication 1, dans lequel le taux d'élution (R) est déterminé conformément à la formule $R = 0{,}0493 \ln (A_{C60+}/ A_{Etotal} \times 100) + 1{,}1746$.

**3.** Procédé selon la revendication 2, comprenant en outre :

la détermination d'une pente d'une ligne de non élution conformément à la formule pente = $(0 - A_{C60})/(C_{MAX} - 60)$ ; et
la détermination d'au moins une fraction d'aire pour un nombre de carbones ($C_{NI}$) conformément à la formule $A_{CNI} = A_{C60} + \text{pente} \times (C_{NI} - 60)$.

**4.** Procédé selon la revendication 3, comprenant en outre la détermination d'une fraction d'aire $A_{CNI}$ pour chaque nombre de carbones ($C_{NI}$) entre $C_{60}$ et $C_{MAX}$.

**5.** Procédé selon la revendication 4, comprenant en outre la détermination d'une fraction molaire pour chaque nombre de carbones ($C_{NI}$) entre $C_{60}$ et $C_{MAX}$.

**6.** Procédé selon la revendication 5, comprenant en outre la sommation de la fraction de poids moléculaire pour chaque nombre de carbones ($C_{NI}$) entre $C_{60}$ et $C_{MAX}$ de manière à déterminer un poids moléculaire de $C_{60+}$ ($MW_{C60+}$) du pétrole lourd.

**7.** Procédé selon la revendication 6, comprenant en outre la sommation de la fraction de poids moléculaire pour chaque nombre de carbones ($C_{NI}$) entre $C_{90}$ et $C_{MAX}$ de manière à déterminer un poids moléculaire ($MW_{C90+}$) pour des nombres de carbone supérieurs et égaux à 90 ($C_{90+}$) pour le pétrole lourd.

**8.** Procédé selon la revendication 5, comprenant en outre la sommation de la fraction de poids moléculaire pour chaque nombre de carbones ($C_{NI}$) entre $C_{MIN}$ et $C_{MAX}$ de manière à déterminer un poids moléculaire (MW) du pétrole lourd.

**9.** Procédé selon la revendication 8, dans lequel $C_{MIN}$ est égal à $C_3$.

**10.** Procédé selon la revendication 2, dans lequel $A_{C60+}/A_{Etotal} \times 100$ est entre 1 pourcent et 100 pourcents, de préférence entre 2 pourcents et 40 pourcents ou de façon davantage préférable, entre 2 pourcents et 30 pourcents.

**11.** Procédé selon la revendication 5, dans lequel les fractions molaires sont utilisées de manière à obtenir des propriétés matérielles pour le pétrole lourd pour la conception d'un équipement pour convoyer ou traiter le pétrole lourd.

**12.** Procédé selon la revendication 7, dans lequel le $MW_{C60+}$ et le $MW_{C90+}$ sont fournis en tant qu'entrées à un modèle numérique de manière à obtenir des propriétés matérielles de pétrole lourd pour simuler une récupération de pétrole lourd à partir d'un réservoir de pétrole lourd qui contient le pétrole lourd.

**13.** Procédé selon la revendication 8, dans lequel le poids moléculaire (MW) est utilisé de manière à obtenir des propriétés matérielles pour le pétrole lourd pour la conception d'un équipement pour convoyer ou traiter le pétrole lourd.

**14.** Support lisible par ordinateur comportant un code lisible par ordinateur intégré en son sein, dans lequel le code lisible par ordinateur, lorsqu'il est exécuté par un processeur d'un dispositif informatique, met en oeuvre le procédé selon l'une quelconque des revendications 1 à 13.

**15.** Appareil agencé de manière à déterminer une correction de fraction supplémentaire pour du pétrole lourd, l'appareil étant configuré de manière à réaliser le procédé selon l'une quelconque des revendications 1 à 13.

FIG.1

FIG.2

FIG.3

EP 2 863 216 B1

| Carbon Number | Molecular Weight | Area | Mass Fraction | Moles | Mole Fraction | MW | Mole Fraction | MW |
|---|---|---|---|---|---|---|---|---|
| AC60 | 79500 | | | | | | | |
| AC60+ | 2544000 | | | | | | | |
| CN | MW | | | | C60+ | C60+ | C90+ | C90+ |
| 60 | 843.62 | 79500 | 0.0313 | 3.70E-05 | 0.0406 | 34.2877 | | |
| 61 | 857.64 | 78238.095 | 0.0308 | 3.59E-05 | 0.0393 | 33.7434 | | |
| 62 | 871.67 | 76976.19 | 0.0303 | 3.47E-05 | 0.0381 | 33.1992 | | |
| 63 | 885.7 | 75714.285 | 0.0298 | 3.36E-05 | 0.0369 | 32.6549 | | |
| 64 | 899.72 | 74452.38 | 0.0293 | 3.25E-05 | 0.0357 | 32.1107 | | |
| 65 | 913.75 | 73190.475 | 0.0288 | 3.15E-05 | 0.0345 | 31.5664 | | |
| 66 | 927.78 | 71928.57 | 0.0283 | 3.05E-05 | 0.0334 | 31.0222 | | |
| 67 | 941.8 | 70666.665 | 0.0278 | 2.95E-05 | 0.0324 | 30.4779 | | |
| 68 | 955.83 | 69404.76 | 0.0273 | 2.85E-05 | 0.0313 | 29.9337 | | |
| 69 | 969.86 | 68142.855 | 0.0268 | 2.76E-05 | 0.0303 | 29.3894 | | |
| 70 | 983.88 | 66880.95 | 0.0263 | 2.67E-05 | 0.0293 | 28.8452 | | |
| 71 | 997.91 | 65619.045 | 0.0258 | 2.58E-05 | 0.0284 | 28.3009 | | |
| 72 | 1011.94 | 64357.14 | 0.0253 | 2.50E-05 | 0.0274 | 27.7567 | | |
| 73 | 1025.96 | 63095.235 | 0.0248 | 2.42E-05 | 0.0265 | 27.2124 | | |
| 74 | 1039.99 | 61833.33 | 0.0243 | 2.34E-05 | 0.0256 | 26.6682 | | |
| 75 | 1054.02 | 60571.425 | 0.0238 | 2.26E-05 | 0.0248 | 26.1239 | | |
| 76 | 1068.04 | 59309.52 | 0.0233 | 2.18E-05 | 0.024 | 25.5797 | | |
| 77 | 1082.07 | 58047.615 | 0.0228 | 2.11E-05 | 0.0231 | 25.0354 | | |
| 78 | 1096.1 | 56785.71 | 0.0223 | 2.04E-05 | 0.0223 | 24.4912 | | |
| 79 | 1110.12 | 55523.805 | 0.0218 | 1.97E-05 | 0.0216 | 23.9469 | | |

FIG.4A

| | | | | | | | | |
|---|---|---|---|---|---|---|---|---|
| 80 | 1124.15 | 54261.9 | 0.0213 | 1.90E-05 | 0.0208 | 23.4027 | | |
| 81 | 1138.18 | 52999.995 | 0.0208 | 1.83E-05 | 0.0201 | 22.8584 | | |
| 82 | 1152.2 | 51738.09 | 0.0203 | 1.77E-05 | 0.0194 | 22.3142 | | |
| 83 | 1166.23 | 50476.185 | 0.0198 | 1.70E-05 | 0.0187 | 21.7699 | | |
| 84 | 1180.26 | 49214.28 | 0.0193 | 1.64E-05 | 0.018 | 21.2257 | | |
| 85 | 1194.28 | 47952.375 | 0.0188 | 1.58E-05 | 0.0173 | 20.6814 | | |
| 86 | 1208.31 | 46690.47 | 0.0184 | 1.52E-05 | 0.0167 | 20.1372 | | |
| 87 | 1222.34 | 45428.565 | 0.0179 | 1.46E-05 | 0.016 | 19.5929 | | |
| 88 | 1236.36 | 44166.66 | 0.0174 | 1.40E-05 | 0.0154 | 19.0487 | | |
| 89 | 1250.39 | 42904.755 | 0.0169 | 1.35E-05 | 0.0148 | 18.5044 | | |
| 90 | 1264.42 | 41642.85 | 0.0164 | 1.29E-05 | 0.0142 | 17.9602 | 0.0654 | 82.6879 |
| 91 | 1278.44 | 40380.945 | 0.0159 | 1.24E-05 | 0.0136 | 17.416 | 0.0627 | 80.1822 |
| 92 | 1292.47 | 39119.04 | 0.0154 | 1.19E-05 | 0.0131 | 16.8717 | 0.0601 | 77.6765 |
| 93 | 1306.5 | 37857.135 | 0.0149 | 1.14E-05 | 0.0125 | 16.3275 | 0.0575 | 75.1708 |
| 94 | 1320.52 | 36595.23 | 0.0144 | 1.09E-05 | 0.012 | 15.7832 | 0.055 | 72.6651 |
| 95 | 1334.55 | 35333.325 | 0.0139 | 1.04E-05 | 0.0114 | 15.239 | 0.0526 | 70.1594 |
| 96 | 1348.58 | 34071.42 | 0.0134 | 9.93E-06 | 0.0109 | 14.6947 | 0.0502 | 67.6537 |
| 97 | 1362.6 | 32809.515 | 0.0129 | 9.46E-06 | 0.0104 | 14.1505 | 0.0478 | 65.148 |
| 98 | 1376.63 | 31547.61 | 0.0124 | 9.01E-06 | 0.0099 | 13.6062 | 0.0455 | 62.6423 |
| 99 | 1390.66 | 30285.705 | 0.0119 | 8.56E-06 | 0.0094 | 13.062 | 0.0432 | 60.1366 |
| 100 | 1404.68 | 29023.8 | 0.0114 | 8.12E-06 | 0.0089 | 12.5177 | 0.041 | 57.6309 |
| 101 | 1418.71 | 27761.895 | 0.0109 | 7.69E-06 | 0.0084 | 11.9735 | 0.0389 | 55.1253 |
| 102 | 1432.74 | 26499.99 | 0.0104 | 7.27E-06 | 0.008 | 11.4292 | 0.0367 | 52.6196 |
| 103 | 1446.76 | 25238.085 | 0.0099 | 6.86E-06 | 0.0075 | 10.885 | 0.0346 | 50.1139 |

FIG.4B

| | | | | | | | |
|---|---|---|---|---|---|---|---|
| 104 | 1460.79 | 23976.18 | 0.0094 | 6.45E-06 | 0.0071 | 10.3407 | 0.0326 | 47.6082 |
| 105 | 1474.82 | 22714.275 | 0.0089 | 6.05E-06 | 0.0066 | 9.7965 | 0.0306 | 45.1025 |
| 106 | 1488.84 | 21452.37 | 0.0084 | 5.66E-06 | 0.0062 | 9.2522 | 0.0286 | 42.5968 |
| 107 | 1502.87 | 20190.465 | 0.0079 | 5.28E-06 | 0.0058 | 8.708 | 0.0267 | 40.0911 |
| 108 | 1516.9 | 18928.56 | 0.0074 | 4.91E-06 | 0.0054 | 8.1637 | 0.0248 | 37.5854 |
| 109 | 1530.92 | 17666.655 | 0.0069 | 4.54E-06 | 0.005 | 7.6195 | 0.0229 | 35.0797 |
| 110 | 1544.95 | 16404.75 | 0.0064 | 4.17E-06 | 0.0046 | 7.0752 | 0.0211 | 32.574 |
| 111 | 1558.98 | 15142.845 | 0.006 | 3.82E-06 | 0.0042 | 6.531 | 0.0193 | 30.0683 |
| 112 | 1573 | 13880.94 | 0.0055 | 3.47E-06 | 0.0038 | 5.9867 | 0.0175 | 27.5626 |
| 113 | 1587.03 | 12619.035 | 0.005 | 3.13E-06 | 0.0034 | 5.4425 | 0.0158 | 25.0569 |
| 114 | 1601.06 | 11357.13 | 0.0045 | 2.79E-06 | 0.0031 | 4.8982 | 0.0141 | 22.5512 |
| 115 | 1615.08 | 10095.225 | 0.004 | 2.46E-06 | 0.0027 | 4.354 | 0.0124 | 20.0455 |
| 116 | 1629.11 | 8833.32 | 0.0035 | 2.13E-06 | 0.0023 | 3.8097 | 0.0108 | 17.5398 |
| 117 | 1643.14 | 7571.415 | 0.003 | 1.81E-06 | 0.002 | 3.2655 | 0.0091 | 15.0341 |
| 118 | 1657.16 | 6309.51 | 0.0025 | 1.50E-06 | 0.0016 | 2.7212 | 0.0076 | 12.5284 |
| 119 | 1671.19 | 5047.605 | 0.002 | 1.19E-06 | 0.0013 | 2.177 | 0.006 | 10.0227 |
| 120 | 1685.22 | 3785.7 | 0.0015 | 8.83E-07 | 0.001 | 1.6327 | 0.0045 | 7.5171 |
| 121 | 1699.24 | 2523.795 | 0.001 | 5.84E-07 | 0.0006 | 1.0885 | 0.0029 | 5.0114 |
| 122 | 1713.27 | 1261.89 | 0.0005 | 2.90E-07 | 0.0003 | 0.5442 | 0.0015 | 2.5057 |
| 123 | 1727.3 | 0.015 | 0 | 0.00E+00 | 0 | 0 | 0 | 0 |
| Summations | | 2544000 | 1 | | 1 | 1097.2 | 1 | 1405.69 |
| | | | | | | MW C60+ | | MW C90+ |
| | | | Moles C60+ | 9.11E-04 | | | | |
| | | | Moles C90+ | 1.98E-04 | | | | |

## FIG.4C

EP 2 863 216 B1

**EP 2 863 216 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009073269 A, Steinhecker
- US 2012085149 A, Al-Eid

- WO 2013121204 A, Moorwood

**Non-patent literature cited in the description**

- **ZUO et al.** Plus Fraction Characterization and PVT Data Regression for Reservoir Fluids Near Critical Conditions. *SPE Asia Pacific Oil and Gas Conference and Exhibition, Brisbane,* 16 October 2000